(19) Europäisches Patentamt
European Patent Office
Office européen des brevets

(11) **EP 4 201 329 A1**

(12) **EUROPEAN PATENT APPLICATION**

(43) Date of publication:
**28.06.2023 Bulletin 2023/26**

(21) Application number: **21217683.8**

(22) Date of filing: **24.12.2021**

(51) International Patent Classification (IPC):
**A61B 6/00** (2006.01)    **G01N 23/087** (2018.01)
**H01J 35/00** (2006.01)    **H05G 1/00** (2006.01)

(52) Cooperative Patent Classification (CPC):
**A61B 6/488; A61B 6/405; A61B 6/4241;**
**A61B 6/482; G01N 23/083;** H05G 1/58

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• DAERR, Heiner
  **Eindhoven (NL)**

• SOSSIN, Artur
  **Eindhoven (NL)**
• THRAN, Axel
  **Eindhoven (NL)**
• BRENDEL, Bernhard, Johannes
  **Eindhoven (NL)**
• BONTUS, Claas
  **Eindhoven (NL)**
• ERHARD, Klaus, Alfred
  **Eindhoven (NL)**

(74) Representative: **Philips Intellectual Property &**
**Standards**
**High Tech Campus 52**
**5656 AG Eindhoven (NL)**

(54) **AN IMAGING METHOD AND SYSTEM**

(57)    An imaging method involves selecting both a current level and a kVp level for each of a set of views to be imaged of a region of a subject or object, based on material composition information of the region. In this way, lowest energy can be delivered to achieve a desired signal to noise ratio.

FIG. 2

**Description**

FIELD OF THE INVENTION

The present invention relates to the field of X-Ray imaging, such as imaging using a computed tomography scanner.

BACKGROUND OF THE INVENTION

Computed tomography (CT) is a well-known mechanism for performing imaging of an object or subject. Known CT imaging is performed by transmitting X-rays through the subject or object and reconstructing an image from data obtained from X-ray detector elements at the other side of the subject or object.

Typically an X-ray tube is used to generate the X-rays. An X-ray tube generally operates by using an electron beam generator to generate an electron beam. This electron beam is positioned to be incident upon an X-ray generation surface, which emits X-rays responsive to incident electrons (from the electron beam).

The quality of a computed tomography (CT) image is often degraded by streak artifacts resulting from excessive X-ray quantum noise or photon starvation. When too few photons reach detector elements, strong streaks appear along paths of high X-ray attenuation and an image is only of limited diagnostic value. These photon starvation artifacts for example occur frequently when a pelvis or shoulder is scanned with thin slices.

In the worst case, a patient has to be rescanned at a higher dose or reconstruction has to be performed at a larger slice thickness in order to obtain an acceptable image for diagnosis. This results in a higher dose to the patient, a degraded cross plane resolution, or a reduced patient throughput.

To address this issue, it is known to apply local raw-data filtering, which detects and smooths out the highly noisy part of the raw data. Another approach is to provide planned dose modulation by implementing X-ray tube current modulation with a higher tube current for views with strong attenuation. More advanced countermeasures include pulse width modulation of the X-ray flux using a so-called grid-switch technique.

The approach of dose modulation by adapting the X-ray tube current is slow as the current is controlled via the heating of the cathode filament. It is too slow to boost the X-ray flux for only a small number of consecutive views. Views adjacent to the views with high attenuation will be acquired with a too high dose to reach the needed dose for the high attenuating region. The overall patient dose therefore has to be increased to avoid photon starvation.

A very fast emission current modulation or pulse width modulation of the X-ray flux could be a solution to increase the dose for individual views in order to avoid image degradation by photon starvation. However, these methods require tubes with a dedicated design for the purpose of dose modulation.

There is therefore a need for an imaging approach which provides dose modulation to reduce X-ray exposure, while retaining a desired signal to noise ratio and also preventing photon starvation, but without requiring a highly complex X-ray tube design.

SUMMARY OF THE INVENTION

The invention is defined by the claims.

According to examples in accordance with an aspect of the invention, there is provided an imaging method comprising obtaining anatomic information of a volume to be imaged, the anatomic information providing material composition information, imaging an object by generating views in different angular positions by an x-ray source transmitting radiation to traverse the volume and an energy-resolving detector detecting radiation that traversed the examination region, selecting a current level and a peak kilovoltage, kVp, level for each of a set of views to be imaged based on the material composition information.

This method makes use of anatomic information for X-ray dose modulation planning, in particular including selection of the X-ray tube acceleration voltage (kVp) as well as the current level. A fast kVp switching function is implemented between discrete kVp levels (e.g. 80 kV, 100 kV, 120 kV and 140 kV) for dose modulation in combination with current modulation, in an imaging method based on spectral photon counting. The method enables photon starvation to be avoided, reduces noise-induced bias in the case of high attenuation, and reduces the dose in the case of low attenuation. The views are to be imaged using an energy resolving detector.

The method may comprise performing an initial scan to provide the anatomic information. This initial scan is for example a CT scout scan. Alternatively, the ongoing CT scan may use data from earlier data (earlier slices) of the ongoing scan to provide the anatomic information.

The method may comprise setting a common target signal to noise ratio, SNR, for each of the set of views. By applying a common target, image quality is ensured for all views.

The current levels may then be set to achieve the target signal to noise ratio in each image.

The peak kilovoltage levels, kVp, may be selected from a discrete set of kVp levels.

The method may further comprise adapting the selected current levels in dependence on the resulting current modulation between different views such that the current modulation is implementable in practice. Thus, an optimal derived current modulation scheme may require modification to enable it to be implemented in practice.

The selecting of the kVp level may then take account of the adaptation of the current levels that has been made, so that the desired signal to noise ratio is still achieved.

The method may comprise, for each view, determining a likelihood of photon starvation in the image. Thus, the dose modulation may also aim to prevent photon starvation. For example, selecting a kVp level may comprise determining a minimal kVp level from the discrete set of kVp levels based on the likelihood of photon starvation. This minimal kVp level then avoids photon starvation at the particular current setting that has been determined.

Selecting a kVp level may then comprise determining an optimal kVp level from a subset of the discrete set, the subset comprising the minimal kVp level and higher, based on the selected current level. Thus, the kVp level is chosen from those kVp levels which will prevent photon starvation.

The optimal kVp level for example comprises the lowest kVp level to achieve the target signal to noise ratio. Thus, the kVp level is chosen as the lowest kVp level which can be selected while preventing photon starvation but still achieving the desired signal to noise ratio, based on the (possibly adapted) current level that has been selected.

The method preferably further comprises delivering X-ray energy to a subject or object being imaged and obtaining images for each of the set of views using the selected kVp level and current modulation. Thus, the method involves setting and applying the dose modulation settings.

The invention also provides a computer program comprising computer program code which is adapted, when said program is run on a computer, to implement the method defined above.

The invention also provides an electron beam controller configured to control an electron beam generator for an X-ray tube of a computed tomography or C-arm scanner having an energy resolving X-ray detector, wherein the electron beam controller is adapted to:

> select a current level for each of a set of views based on material composition information of a volume to be imaged; and
>
> select a peak kilovoltage, kVp, level from a discrete set of kVp levels for each of the set of views to be imaged.

The controller is for example further adapted to control the electron beam generator to perform an initial scan to provide the anatomic information.

The controller is for example further adapted to:

> set a common target signal to noise ratio for each of the set of views;
>
> select the current levels to achieve the target signal to noise ratio in each image;
>
> determining a likelihood of photon starvation in the image; and
>
> select the kVp level by determining a minimal kVp level from the discrete set of kVp levels based on the likelihood of photon starvation, and selecting an optimal kVp level from the subset of the discrete set, the subset comprising the minimal kVp level and higher, based on the selected current level.

The invention also provides a computed tomography scanner comprising:

> the electron beam controller defined above;
>
> an electron beam generator configured to generate an electron beam usable for generating X-rays; and
>
> an energy resolving X-ray detector.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Figure 1 is used to explain the basic operation of an X-ray tube;
>
> Figure 2 shows the primary energy spectra for different acceleration voltage levels;
>
> Figure 3 shows a set of three imaging views with different kVp levels at different angular positions of the X-ray source;
>
> Figure 4 shows the geometry of a phantom to simulate the impact of kVp-based dose modulation, showing ideal iodine and water images.

Figure 5 shows iodine and water images with dose modulation;

Figure 6 shows the same images as in Figure 5 but with a smaller window to show artifacts;

Figure 7 shows a simulation of a water phantom without dose modulation at 120 kVp without noise;

Figure 8 shows an imaging method; and

Figure 9 schematically illustrates a system including an imaging system such as a CT scanner.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an imaging method which involves selecting both a current level and a kVp level for each of a set of views to be imaged of a region of a subject or object, based on material composition information of the region. In this way, a lowest X-ray patient dose can be delivered to achieve a desired signal to noise ratio while avoiding photon starvation.

The invention is of particular interest for control of an X-ray source used for a computed tomography scanner, and finds particular use in the medical industry, as well as other industries that employ CT scanners (e.g. the archaeological industry, security industries and so on). The invention may also be used in a C-arm scanner.

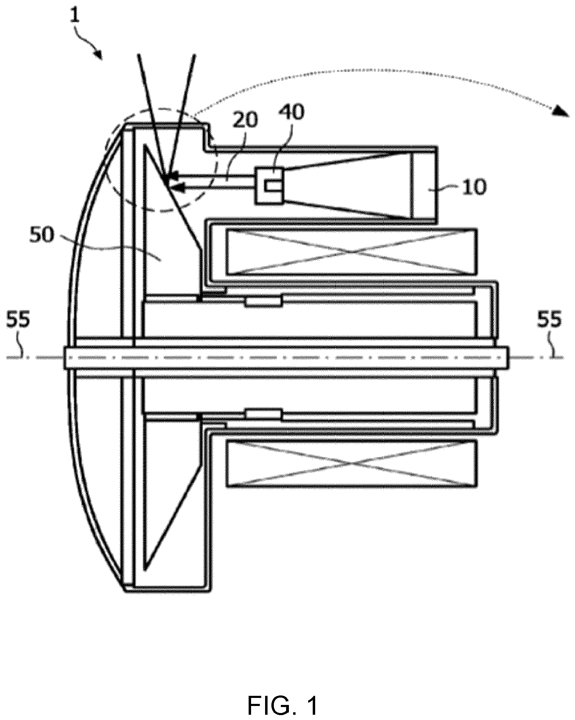

Figures 1 shows an example of an X-ray tube 1, for use in a computed tomography (CT) scanner, for the purposes of improved contextual understanding.

Figure 1 illustrates a cross sectional view of the X-ray tube 1, sometimes labelled an "X-ray exposure tube", for X-ray generation. The X-ray tube comprises a housing, into which a pivoted or rotatable anode 50 is provided, which anode rotates around a rotational axis 55. The rotation of the rotatable anode is controlled by a rotating mechanism (not shown), the operation of which is readily apparent to the skilled person.

The X-ray tube 1 comprises an electron beam generator 10 (alternatively labelled an electron beam source), which generator is capable of emitting an electron beam 20. The electron beam may be steered (e.g. positioned) by an electron beam steering module 40. The electron beam hits the surface of the anode 50 and owing to its high impact energy generates an electromagnetic ray, in particular an X-ray, which may be emitted via a window on e.g. the lateral side of the X-ray tube. X-rays emitted via the window are subsequently detectable by X-rays sensors of the computed tomography scanner.

It will be apparent that the electron beam generator thereby acts as a cathode for providing an electron beam between the cathode and the anode 50.

The voltage level used by the X-ray tube may be represented by the peak kilovoltage (kVp), which is the maximum high voltage in which the electron beam operates when creating the electron beam. The peak kilovoltage corresponds to the highest kinetic energy of the electrons in an electron beam that is incident upon the anode, and is proportional to the maximum energy of the resulting X-ray emission spectrum. The X-ray tube is also operated at a particular tube current determining the velocity and number of electrons within the electron beam.

Spectral imaging is becoming an increasingly popular imaging methodology of computed tomography. Spectral imaging is performed by detecting X-rays passed through the object of interest at different energy levels. There are different techniques to accomplish this. The energy discrimination can be done at the detector level using so-called energy resolving detectors like in multi energy bin photon counting detectors and/or by switching a total energy of emitted X-rays between a higher energy level and a lower energy level. This results in different spectra being provided to a subject. One method for performing spectral imaging is to switch a voltage level used to generate the electron beam between a first voltage level and a second voltage level. This is commonly known as kVp switching.

By way of example, it may typically take around 100 $\mu$s to increase the voltage by 60kV (e.g. from 80kVp to 140kVp) and around 200 $\mu$s to 300 $\mu$s to decrease the voltage.

The integration period (the time between images) is for example as short as 150 $\mu$s in some systems, with the imaging system for example performing a revolution in 0.36 seconds for 2400 images per revolution. Thus, fast kVp switching, for example meaning that a kVp level change of 60kVp can be accomplished in about 50$\mu$s, may be used. However, it may only be possible to switch with smaller kVp increments between individual successive views.

Different voltage levels will result in different photon flux. The photon flux f and hence the X-ray dose, is proportional to the 2.5th power of the acceleration voltage kVp , i.e. $f \sim kVp^{2.5}$.

Correspondingly, a change of the kVp level from 120 kV to 140 kV increases the flux by a factor of about 1.5 when the X-ray tube current is constant. Typically, the X-ray tube current also increases by 10% or 20% when the kVp level is increased by 20 kV.

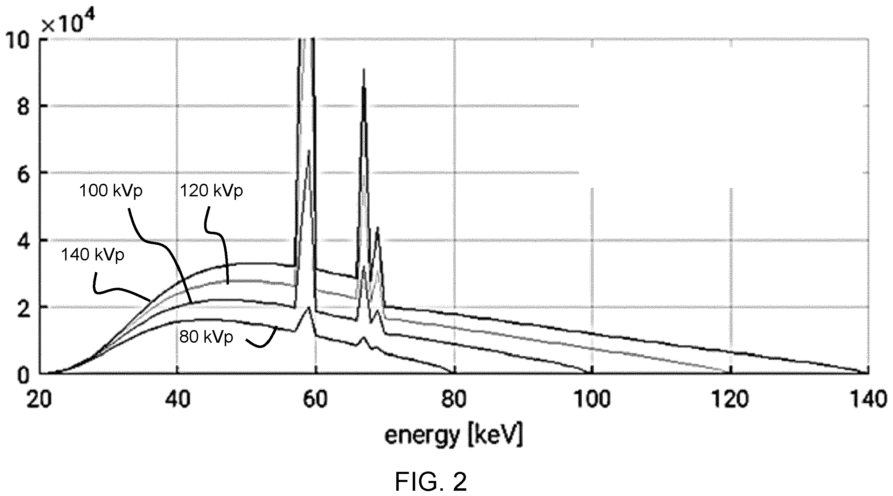

Figure 2 shows the primary energy spectra for different acceleration voltage levels. The air KERMA (the energy released in a unit mass of air) increases strongly with the kVp value. In addition, the effective mean energy of the spectrum shifts to higher energies with increasing kVp and by this the effective attenuation coefficient of the object gets smaller.

As a result, the amount of transmitted photons increases roughly by a factor of 1.3 for a 20 kV higher kVp value at the same entrance dose, for a penetration length of 300 mm of water.

Table 1 below shows the out dose and the photon flux collected after transmission through the subject. The out dose is shown both relative to the KERMA value (by definition measured in air without the patient) and as an absolute value. The table shows values for different kVp levels and for two different tissue types, namely 300mm water ("W") and 300mm water + 30mm bone ("W+B"). The values are all normalized with respect to the values at 80 kVp.

Overall, for a length of 300 mm of water, the delivered dose and the total photon flux after the subject or object approximately double for each increase of 20 kV at a constant X-ray tube current.

The detected signals can thus be boosted immediately by a factor of at least 2. This factor slightly increases in case bone is present.

Table 1

| kVp | outDose divided by KERMA W | outDose W | Photonflux W | outDose divided by KERMA W+B | outDose W+B | Photon flux W+B |
|-----|------------------------------|-----------|--------------|-------------------------------|-------------|------------------|
| 80  | 1    | 1    | 1     | 1    | 1     | 1     |
| 100 | 1.5  | 3    | 3.13  | 2.73 | 6.22  | 5.3   |
| 120 | 1.95 | 5.84 | 6.83  | 5.32 | 20.24 | 15.09 |
| 140 | 2.39 | 9.31 | 12.46 | 8.77 | 48.08 | 31.97 |

The invention makes use of a CT system with fast kVp switching capabilities comprising an energy resolving X-ray detection system such as a multi energy bin photon counting detector. The fast kVp switching requires that the X-ray tube, the generator, the focal spot size control, the current control and detector-tube communication are suitable for fast kVp-switching.

In particular, the invention involves planning of the dose modulation for different views to be imaged.

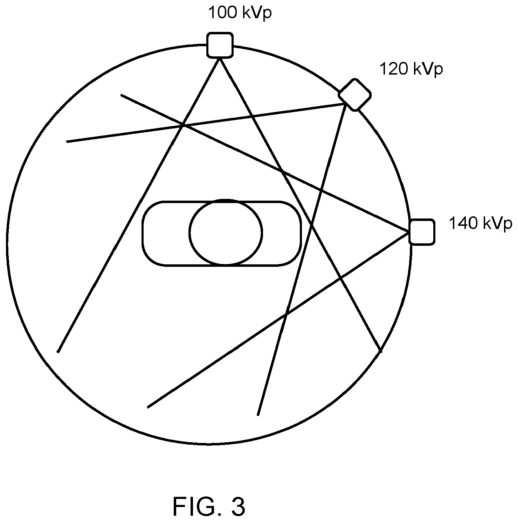

Figure 3 by way of example shows a set of three imaging views with 100 kVp, 120 kVp and 140 kVp at different angular positions of the X-ray source.

An energy resolving X-ray detection system is required when changing the kVp level because it enables to combine path length calculated from view acquired at different kVp levels in one representation/sonogram even though acquired at different kVp levels.

The invention involves planning of the dose modulation for each of the different views by using anatomic information, which is for example obtained from a scout scan using an energy resolving detector such as a multi energy bin photon counting detector. The scout scan may be a 2D scan or a 3D scan. The scout view of a CT scanner is an X-ray projection radiograph by scanning with a fixed fan beam, and is typically used for planning a CT examination.

An example of the possible steps of the method of the invention is now discussed in detail.

Step 1

The spectral data of an un-enhanced scout scan may for example be decomposed into water and bone, based on the attenuation caused by photoelectric absorption (or Compton scatter at higher energies). The patient may then be modelled as a water equivalent ellipsoid based on the water equivalent diameter (WED).

The signal to noise ratio in the images is mainly influenced by the number of detected photons thus by the net attenuation of the object. Therefore, a water equivalent ellipsoid may be fitted to each slice or table position of the scout scan data.

Step 2

A reference kVp level (e.g. 120 kVp) and a target signal to noise ratio, SNR, are selected by the user or may be part of the user-selected protocol. From the material composition information, a desired dose can be calculated for each view to reach the target signal to noise ratio, SNR.

Note that the use of a reference level is optional. It means the kVp values are calculated as deviations from the reference level, but they may be calculated in isolation without the need of a reference level.

Step 3

The user then selects the contrast agent material being used, for example water and iodine, or water and iodine and a further agent with a different K-edge. This step may be used so that the current modulation scheme may be adapted. For example, the use of a contrast agent may require a different current modulation for different directions (e.g. from above or laterally) than would follow from a basic water ellipsoid assumption.

Step 4

A dose modulation, based on current modulation at the reference kVp level, is then calculated for the given material decomposition, thereby to reach the desired dose resulting from the material composition analysis.

This is the same as conventional planning of the current modulation scheme. Note that a current modulation scheme may involve a fixed current for each revolution or it may involve a current which varies around the scan revolution.

It can be assumed that for the reference kVp level, the amount of detected photon counts per current and the water equivalent diameter (WED) is known.

The purpose of the current modulation is to enable each view to have the same signal to noise ratio. Thus, there is a target signal to noise ratio, SNR, for each image.

The number of needed photons nPh (or needed tube current) to reach the target SNR for each view is determined. This may be achieved by using a look-up table from measurements or based on system simulations or by performing a Cramer-Rao Lower Bound, CRLB, analysis.

The resulting current modulation might not be realizable for some slices as the WED ellipsoid provides a too large aspect ratio and the current modulation is too slow to follow the required current changes. In such a case, the current modulation is adjusted to be realizable.

This adjustment limits the increase and decrease of current between views, and is calculated from the start of the imaging sequence to the end. Thus, a smooth and realizable current modulation scheme is created. This adjusted current modulation scheme based on a smooth water ellipsoid fitted to the scout scan data may include views with a too high dose resulting in a higher SNR at the target and may include views with a too low dose resulting in a lower SNR or even photon starvation.

Step 5

As explained above, image degradation is caused by photon starvation. The dosing method thus takes account of the likelihood for photon starvation.

The likelihood for photon starvation for each view and kVp level (80kVp, 100 kVp, 120 kVp, 140 kVp) may be expressed as:

$$P_{starv}(view, kVp)$$

This probability may be calculated based on the actual material decomposition of the scout scan and based on the selected tube current (including tube current changes due to kVp level changes). This may involve evaluating for each detector pixel d and for each view v the probability for counting less than $n_t$ counts in total ($n_t$ = 0,1,2..5) given by the Poisson distribution:

$$P_{d,v}(n_t) = \sum_{k=0}^{n_t-1} \frac{\lambda^k}{k!} e^{-\lambda}$$

Photon starvation is likely to occur if the average over several adjacent detector pixels (e.g. 10) exceeds a given threshold T (e.g. 0.15) (corresponding to the case that a fraction T of pixels count less than $n_t$ photons). This average is represented as:

$$\overline{P_{d,v}(n_t)}$$

Step 6

For each view, a minimal allowed kVp level from the discrete set is determined based on the evaluation of the likelihood for photon starvation using the threshold T. For views which satisfy:

$$P_{starv}(view, kVp) < T$$

a minimal allowed kVp is set to the lowest possible kVp value (e.g. 80 kVp) out of the satisfying kVp levels.

Step 7

After the current modulation scheme has been derived (and adapted to make it realizable), a kVp switching scheme is derived. For each view, the optimal kVp level of the remaining allowed kVp levels (i.e. a subset of the discrete set, from the minimal allowed kVp level up to the maximum) is determined based on the selected tube current and material decomposition i.e. the real bone-water path length data from the scout scan.

An optimal kVp level is one for which more than a fraction X (e.g. 0.8) of the pixels masked by the patient have photon counts larger than the number needed to reach the target SNR for the given kVp-level. The lowest kVp level is selected as it provides the lowest dose.

This results in a kVp modulation pattern between views. The imaging method thus makes use of fast kVp switching. This fast kVp-switching provides a new degree of freedom for dose modulation. Here, fast kVp switching corresponds to a transition time from low to high kVp (or vice versa) within an integration period of about 100 μs to 200 μs as explained above.

Step 8

This resulting kVp modulation pattern might however not be feasible as it might be the case that kVp changes occur from view to view and the change in the kVp level cannot be implemented quickly enough to settle between successive views.

Therefore, the system may allow a change of the kVp-level to be implemented only after N (N=10,20,50 for example) views are acquired at a given kVp-level. Thus, the modulation pattern is effectively applied at the level of sets of N views rather than to individual views, so that kVp level is selected so that for all views of the set, the signal to noise ratio condition is met.

The kVp modulation pattern may be modified to meet this requirement in various ways.

One approach is to ensure that the number of adjacent views with a minimal required kVp level is equal or larger than N (to avoid photon starvation).

Another approach is to apply median filtering to the kVp pattern with a kernel size of 2*N+1

This dose modulation planning system has been tested by noiseless simulations of a multi energy bin photon counting CT system to identify artifacts. Photon counts for a water ellipsoid with two iodine and two calcium inserts were simulated.

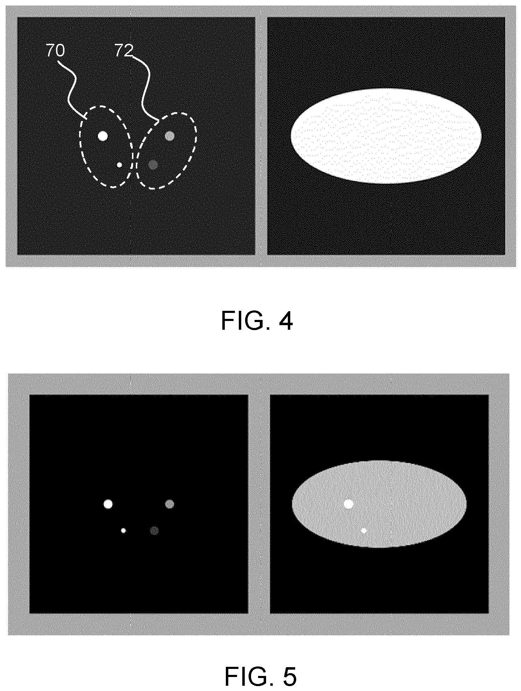

Figure 4 shows the geometry of a phantom to simulate the impact of kVp-based dose modulation. A water-iodine material decomposition was performed. The resulting water and iodine images are presented in Figures 5 to 7. In each case, calcium is visible in both images.

The left image in Figure 4 shows an ideal iodine image showing calcium (bone) inserts in region 70 with two different sizes on the left and iodine inserts in region 72 with two different concentrations on the right.

The right image shows an ideal water image showing the water ellipsoid. The bone inserts appear also in this image since bone is represented by certain concentrations of water and iodine in a water-iodine material decomposition.

Figure 5 shows corresponding iodine and water images with dose modulation (100 kVp, 120 kVp, 130 kVp) and normal level and window settings (water: level 50 HU; window 500 HU). No image artifacts are visible.

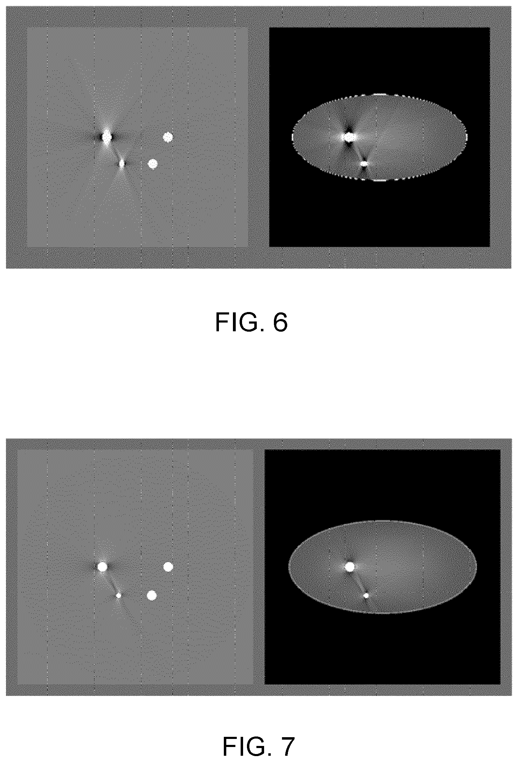

Figure 6 shows the same images as in Figure 5 but with a smaller window (+-5HU in the water image on the right) to show artifacts.

Figure 7 shows a simulation of a water phantom without dose modulation at 120 kVp without noise. A small CT window is used (+-5HU in the water image) to show artifacts as in Figure 7.

The images thus show that artifacts around calcium inserts are intensified by the dose modulation (as seen by comparing Figures 5 and 6). The artifacts around the calcium insert increased slightly from -3 HU to -8 HU in water. This increased artefact level is acceptable as it is outweighed by the reduced photon starvation artefacts and a reduction of patient dose achievable with this method. As expected, the iodine inserts are free of artifacts in all images.

The CT scanner for example uses a multi-bin photon counting system. The optimal setting of the thresholds in such a bin counting system depends on the primary spectrum. The switching of kVp may also require a fast change of the selected thresholds.

Thresholds at K-edge energies will never be modified. The material decomposition has to use the correct spectrum for the kVp setting of each acquired projection, as well as the correct thresholds. It is also possible to use constant thresholds for the whole scan, independent of the applied kVp settings, which might result in (slight) loss of SNR in the final material images.

For a multi-bin photon counting system (e.g. 4, 5 or more bins) it might not be necessary to transfer all bin data but to sum up data of selected bins to reduce the number of bins (e.g. 2 or 3 depending on the imaging task) an thus the amount of data to be transferred. The selection of the bins to be combined may depend on the selected kVp-level.

There are various alternatives to the steps of the method explained above.

In step 3, instead of letting requiring the user to choose a material basis, the system may be optimized for certain thresholds such as a-three material decomposition. This fits better to the philosophy of having spectral analysis "always on".

The method described above makes use of a scout scan for the initial material decomposition information. In an alternative implementation, the dose modulation is not based on a scout scan. Instead, the dose may be modulated just-in-time as measurements are taken. The measurements in this case need to be evaluated fast enough with respect to the minimum number of photons reaching the detector. If this number is too small, the dose must be increased. If this number is too large, the dose must be reduced.

Since kVp switching allows for a fast reaction, this feedback loop is possible.

In an alternative implementation, the dose modulation may be a combination of a plan obtained from a scout scan and a fast feedback loop based on measurements as explained above.

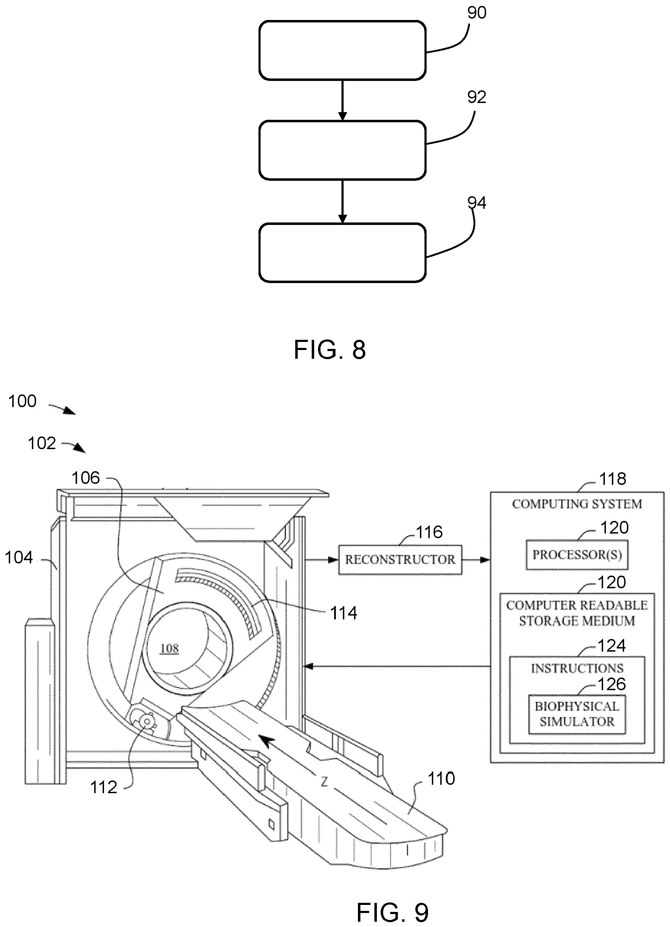

Figure 8 shows an imaging method, comprising, in step 90, obtaining anatomic information of a volume to be imaged, the anatomic information providing material composition information. A current level is set in step 92 for each of a set of views to be imaged based on the material composition information. A peak kilovoltage, kVp, level is also selected from a discrete set of kVp levels for each of the set of views to be imaged. Anatomic information is thus used for X-ray dose modulation planning, in particular including selection of the X-ray tube acceleration voltage (kVp) as well as the current level.

For the sake of improved contextual understanding, Figure 9 schematically illustrates a system 100 including an imaging system 102 such as a CT scanner. The imaging system 102 includes a generally stationary gantry 104 and a rotating gantry 106, which is rotatably supported by the stationary gantry 104 and rotates around an examination region 108 about a z-axis. A subject support 110, such as a couch, supports an object or subject in the examination region 108.

A radiation source 112, such as an x-ray tube, is rotatably supported by the rotating gantry 106, rotates with the rotating gantry 106, and emits radiation that traverses the examination region 108. In the context of the present invention, the radiation source 112 includes an x-ray tube configured to switch between a discrete set of different emission voltages during scanning as well as tube currents. The radiation source 112 may include two or more x-ray tubes configured to emit radiation having different mean spectra.

A radiation sensitive detector array 114 subtends an angular arc opposite the radiation source 112 across the examination region 108. The radiation sensitive detector array 114 detects radiation traversing the examination region 108 and generates an electrical signal(s) (projection data) indicative thereof. Where the radiation source 112 includes a single broad spectrum x-ray tube, the radiation sensitive detector array 112 includes energy-resolving detectors (e.g., direct conversion photon counting detectors, at least two sets of scintillators with different spectral sensitivities (multi-layer), etc.). With kVp switching and multi-tube configurations, the detector array 114 can include single layer detectors, direct conversion photon counting detectors, and/or multi-layer detectors. The direct conversion photon counting detectors may include a conversion material such as CdTe, CdZnTe, Si, Ge, GaAs, or other direct conversion material.

A reconstructor 116 receives spectral projection data from the detector array 114 and reconstructs spectral volumetric image data such as sCCTA image data, a high-energy image, a low energy image, a photoelectric image, a Compton scatter image, an iodine image, a calcium image, a virtual non-contrast image, a bone image, a soft tissue image, and/or other basis material image. The reconstructor 116 can also reconstruct non-spectral volumetric image data, e.g., by combining spectral projection data and/or spectral volumetric image data. Generally, the spectral projection data and/or spectral volumetric image data will include data for at least two different energies and/or energy ranges.

A computing system 118 serves as an operator console. The console 118 includes a human readable output device such as a monitor and an input device such as a keyboard, mouse, etc. Software resident on the console 118 allows the operator to interact with and/or operate the scanner 102 via a graphical user interface (GUI) or otherwise. The console 118 further includes a processor 120 (e.g., a microprocessor, a controller, a central processing unit, etc.) and a computer readable storage medium 122, which excludes non-transitory medium, and includes transitory medium such as a physical memory device, etc. The processor 120 implements the control of the overall system 100 to implement the imaging method of the invention.

The computer readable storage medium 122 includes instructions 124 for carrying out one or more tasks using the processor. The processor 120 is configured to execute the instructions 124. The processor 120 may additionally be configured to execute one or more computer readable instructions carried by a carrier wave, a signal and/or other transitory medium. In a variation, the processor 120 and the computer readable storage medium 122 are part of another computing system, which is separate from the computing system 118.

Examples of components that may be employed by the computing system include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

## Claims

1. An imaging method comprising:

   obtaining (90) anatomic information of a volume to be imaged, the anatomic information providing material composition information;
   imaging an object by generating views in different angular positions by an x-ray source transmitting radiation to traverse the volume and an energy-resolving detector detecting radiation that traversed the examination region (108),

   **characterized in that** the method further comprises
   selecting (92, 94) a current level and a peak kilovoltage, kVp, level for each of a set of views based on the material composition information.

2. The method of claim 1, comprising performing an initial scan or earlier data from the ongoing scan to provide the anatomic information.

3. The method of claim 1 or 2, comprising setting a common target signal to noise ratio for each of the set of views and selecting current levels to achieve the target signal to noise ratio in each image.

4. The method of claim 1, wherein the peak kilovoltage levels, kVp, are selected from a discrete set of kVp levels.

5. The method of claim 3, comprising adapting the selected current levels in dependence on the resulting current modulation between different views such that the current modulation is implementable in practice.

6. The method of any one of claims 1 to 5, comprising, for each view, determining a likelihood of photon starvation in the image.

7. The method of claim 6, wherein selecting a kVp level comprises determining a minimal kVp level from the discrete set of kVp levels based on the likelihood of photon starvation.

8. The method of claim 7, wherein selecting a kVp level comprises determining an optimal kVp level from the subset of the discrete set, the subset comprising the minimal kVp level and higher, based on the selected current level.

9. The method of claim 8, wherein the optimal kVp level comprises the lowest kVp level to achieve a target signal to noise ratio.

10. The method of any one of claims 1 to 9, further comprising delivering X-ray energy to a subject or object being imaged and obtaining images for each of the set of views using the selected kVp level and current modulation.

11. A computer program comprising computer program code which is adapted, when said program is run on a computer, to implement the method of any one of claims 1 to 10.

12. An electron beam controller configured to control an electron beam generator for an X-ray tube of a computed tomography scanner having an energy resolving X-ray detector, wherein the electron beam controller is adapted to:

   select a current level for each of a set of views based on material composition information of a volume to be imaged; and
   selecting a peak kilovoltage, kVp, level from a discrete set of kVp levels for each of the set of views to be imaged.

13. The controller of claim 12, wherein the controller is further adapted to control the electron beam generator to perform an initial scan to provide the anatomic information.

14. The controller of claim 12 or 13, wherein the controller is further adapted to:

   set a common target signal to noise ratio for each of the set of views;
   select the current levels to achieve the target signal to noise ratio in each image;
   determining a likelihood of photon starvation in the image; and
   select the kVp level by determining a minimal kVp level from the discrete set of kVp levels based on the likelihood of photon starvation. and selecting an optimal kVp level from the subset of the discrete set, the subset comprising the minimal kVp level and higher, based on the selected current level.

15. A computed tomography scanner comprising:

   the electron beam controller of any one of claims 12 to 14;
   an electron beam generator configured to generate an electron beam usable for generating X-rays; and
   an energy resolving X-ray detector.

FIG. 1

FIG. 2

FIG. 3

FIG. 4

FIG. 5

FIG. 6

FIG. 7

FIG. 8

FIG. 9

Europäisches
Patentamt

European
Patent Office

Office européen
des brevets

# EUROPEAN SEARCH REPORT

**Application Number**

**EP 21 21 7683**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/144764 A1 (NISHIDE AKIHIKO [JP] ET AL) 19 June 2008 (2008-06-19) | 1,2,4,5, 10-13,15 | INV.<br>A61B6/00 |
| Y | * paragraphs [0030], [0132], [0074], [0075], [0117], [0086] * | 3,14 | G01N23/087<br>H01J35/00<br>H05G1/00 |
| X | US 2020/054302 A1 (SCHAEFER DIRK [DE] ET AL) 20 February 2020 (2020-02-20) | 1,2,10, 11 | |
| Y | * paragraphs [0044], [0027], [0049] * | 3 | |
| X | US 2020/093451 A1 (NETT BRIAN EDWARD [US] ET AL) 26 March 2020 (2020-03-26) | 1,2,10, 11 | |
| Y | * paragraphs [0034], [0047], [0035] * | 3 | |
| X | US 2014/321603 A1 (TAGUCHI HIROKI [JP] ET AL) 30 October 2014 (2014-10-30) | 1,2,6-11 | |
| Y | * paragraphs [0023], [0025], [0026], [0023], [0005] * | 3 | |
| Y | US 2019/142357 A1 (FAN JIAHUA [US] ET AL) 16 May 2019 (2019-05-16)<br>* paragraph [0139] * | 3,14 | |

TECHNICAL FIELDS
SEARCHED (IPC)

A61B
G01N
H05G
H01J

The present search report has been drawn up for all claims

| Place of search | Date of completion of the search | Examiner |
|---|---|---|
| Munich | 16 May 2022 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 7683

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2022

| Patent document cited in search report | | Publication date | Patent family member(s) | | Publication date |
|---|---|---|---|---|---|
| US 2008144764 | A1 | 19-06-2008 | CN | 101229063 A | 30-07-2008 |
| | | | JP | 5389324 B2 | 15-01-2014 |
| | | | JP | 2008148886 A | 03-07-2008 |
| | | | US | 2008144764 A1 | 19-06-2008 |
| US 2020054302 | A1 | 20-02-2020 | CN | 104136938 A | 05-11-2014 |
| | | | EP | 2820449 A2 | 07-01-2015 |
| | | | JP | 5774242 B2 | 09-09-2015 |
| | | | JP | 2015508001 A | 16-03-2015 |
| | | | RU | 2014139064 A | 20-04-2016 |
| | | | US | 2015038839 A1 | 05-02-2015 |
| | | | US | 2020054302 A1 | 20-02-2020 |
| | | | WO | 2013128336 A2 | 06-09-2013 |
| US 2020093451 | A1 | 26-03-2020 | NONE | | |
| US 2014321603 | A1 | 30-10-2014 | CN | 104105445 A | 15-10-2014 |
| | | | JP | 6261915 B2 | 17-01-2018 |
| | | | JP | 2014061286 A | 10-04-2014 |
| | | | US | 2014321603 A1 | 30-10-2014 |
| | | | WO | 2014034888 A1 | 06-03-2014 |
| US 2019142357 | A1 | 16-05-2019 | CN | 109770933 A | 21-05-2019 |
| | | | US | 2019142357 A1 | 16-05-2019 |

EPO FORM P0459

For more details about this annex : see Official Journal of the European Patent Office, No. 12/82